# EUROPEAN PATENT APPLICATION

(11) **EP 3 034 081 A1**
(43) Date of publication of application: **22.06.2016**
(21) Application number: 14307093.6
(22) Date of filing: 18.12.2014
(51) Int. Cl.: A61K 31/551, A61P 27/02

(54) **Treatment of an inflammation of the tarsal gland of the eyelid**

(71) Applicant: Neuroptis Biotech, 75008 Paris (FR)
(72) Inventor: Belot, Eric, 75001 PARIS (FR)
(74) Representative: Sekhri, Redha

(57) **Abstract**

The present invention relates to a method for treating an inflammation and/or dilatation of the tarsal gland (or meibomian gland) of the eyelid.

## Description

The present invention relates to a method for treating an inflammation and/or dilatation of the tarsal gland (or meibomian gland) of the eyelid.

Inflammation or dilatation of the tarsal gland may contribute to a number of conditions, including blepharitis. Therefore, there is a need for therapeutic means for treating this inflammation or dilatation.

The present invention relates to a compound selected in the group consisting of ML7 and a functional derivative thereof, for use in a method for the treatment of an inflammation and/or dilatation of the tarsal gland of the eyelid.

ML7, or 1-(5-iodonaphtalene-1-sulfonyl)-1H-hexahydro-1,4-diazepine, was described in Saitoh et al., J. Biol. Chem 1987, 262(16), p. 7796. It is derived from the ML9 molecule (1-(5-chloro-1-sulfonyl)-1H-hexahydro-1,4-diazepine) and capable of selectively inhibiting the activity of the myosin light chain kinase (or MLCK).

According to the present invention a functional derivative of ML7 is a molecule according to formula (I) 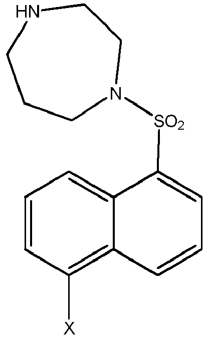 wherein X is a halogen atom.

In a particular embodiment, X is a chlorine atom, and the derivative being ML9.

In a preferred embodiment, the invention implements the use of compound ML7 or ML9, for the treatment of an inflammation and/or dilatation of the tarsal gland of the eyelid.

In a preferred embodiment, the compound administered to the subject in need thereof is ML-7.

In a particular embodiment, the compound is administered in as a pharmaceutical composition, in particular a topical composition for administration to the eye, such as an eye drop, in particular a multidose eye drop, comprising said compound and a pharmaceutically acceptable carrier. A particular topical composition for administration to the eye comprises
- between 0,001 and 0,5 weight % of the compound;
- between 0,1 and 10 weight % of cyclodextrine; and
- between 0,01 and 0,5 weight % of hydroxypropylmethylcellulose, PVP hydroxyethylcellulose or PVA.

The composition may be completed to 100% with a pharmaceutically acceptable excipient such as water.

In a particular embodiment, the cyclodextrine is hydroxypropyl beta-cyclodextrine.

In a further particular embodiment, the pH of the composition is comprised between 6 and 7.5, in particular between 6.8 and 7.2.

The inventors have surprising shown that rabbits which received topical administration of benzalkonium chloride (BAC) showed an absence of inflammation or dilation of the tarsal gland of the eyelid when receiving a compound according to the present invention, as compared to the control group which did not receive said compound.

This surprising finding has implications for the treatment of the inflammation or dilation of the eyelid of a subject in need thereof.

In the context of the present invention, the term "treatment" or "treating" refers to therapy, prevention, or prophylaxis of an inflammation and/or dilatation of the tarsal gland in a subject in need thereof. The treatment may involve the administration of a pharmaceutical composition to the subject having a declared disorder to prevent, cure, delay, reverse, or slow down the progress of the disorder, improving thereby the condition of the subject. A treatment may be also administered to a subject that is either healthy or at risk of developing an inflammation and/or dilatation of the tarsal gland of the eyelid.

In the context of the present invention, the term "subject" refers to a mammal and more particularly a human.

The compound is used according to the invention in an effective amount. The term "effective amount" denotes that amount that is effective in treating an inflammation and/or dilatation of the tarsal gland of the eyelid. Such effective amount may be comprised between 0.001 weight % and 0.5 weight %, for example an amount of 0.1 wt%. The compound may be administered once a day or several times a day. For example, the compound is administered 2 to 3 times per day. In a particular embodiment, the compound is administered for one or more days, for example, for at least one, two, three, four, five, six or seven days. In a specific embodiment, the compound is administered for at least one week.

The present invention is further illustrated with reference to the following examples.

### EXAMPLES

### 1. Materials and methods

The test system consisted of Naive New Zealand White rabbits (total 20 males; 2 to 3 Kg and 4 to 6 months of age at start of BAC pretreatment). Source: Covance Research Products Inc. Denver, USA.

The study design was as follows:

**Table 1 - Experimental Design**

| **Day** | **Test Item** | **Concentration** | **No. of Applications/Day** | **Animal No.** |
|---|---|---|---|---|
| **BAC pretreatment Phase** | | | | |
| 1 to 36 | BAC | 0.1% | 2 | All |
| 37 to 48 | BAC | 0.1% | 4 | All |
| 49 to 60 | BAC | 0.15% | 4 | All |

| **Dose Administration Phase** | | | | |
|---|---|---|---|---|
| 61 to 70 | PBS | 0 | 3 | 1002-1006, 1101 |
| | Placebo | 0 | 3 | 2001-2006 |
| | ML7 | 0.1% | 3 | 3001-3006 |

| | | | | |
|---|---|---|---|---|
| BAC = Benzalconium chloride (BAC) Detailed examination and body weight were performed weekly, morbidity/mortality checks were performed at least twice daily (AM and PM) and cage side observations were performed once daily throughout the BAC pretreatment and dosing phases. | | | | |

### 1.1. BAC pretreatment Phase

All study animals were treated with benzalkonium chloride (BAC) by topical ocular instillation during the pretreatment phase. Measurement of tear production was performed weekly during the pretreatment phase in order to determine when the animal was sufficiently induced.

For the first 5 weeks, 0.1% BAC was administered twice daily (normally separated by at least 6 hours) by ocular instillation (50 µL drop size) to both eyes. Tear production measurements showed an increase in tear production, therefore, the pretreament phase was prolonged. BAC administration was increased to four-times daily from Days 37 to 48. The concentration of BAC was subsequently increased to 0.15%, and administered four-times daily from Days 49 to 60. Animals were administered buprenorphine SR (0.1 mg/kg) twice weekly, starting 2 weeks after the start of BAC administration and throughout the remainder of the study to minimize pain and discomfort due to irritation of the eyelids.

During the pretreatment phase, gross ocular examinations were performed weekly, and ophthalmic examination was performed once prior to the start of BAC administration and on Days 34 and 48.

### 1.2 Dose Administration Phase

The test item (ML7) and the placebo were used. Vials were transferred to the dosing area on wet ice. PBS was dispensed as daily aliquots, which were handled in the same manner as the test item and placebo.

The test and reference items (placebo and PBS) were administered to the appropriate animals three times daily by ocular instillation (equally spaced during a normal working day) from Days 61 to 70. The dose volume was 50 µL/eye/dose. Dose formulations were removed from storage 20-30 minutes prior to dosing to allow them to equilibrate to ambient temperature, and were kept refrigerated or on wet ice between doses. One spare animal remained on study but was not dosed.

During the dose administration phase, gross ocular examinations were performed on Days 61, 63, 65, 67, and 70, prior to the second daily dose. Tear production measurements were performed once prior to dosing start (on Day 61), and on Days 65 and 70. Ophthalmic examination was performed on the third day of dose administration (Day 63) and again prior to necropsy. Images of the eye and eyelids were captured for all animals, when possible.

### 1.3. Terminal Procedures and Histopathology

Due to clinical signs observed during the BAC pretreatment period (skin lesions in the dorsal region), which were unrelated to BAC administration, one animal was euthanized and replaced by a spare. No necropsy or tissues were collected for this animal.

Animals surviving to study termination, including the spare animal, were euthanized on Day 71 by intravenous injection of sodium pentobarbital, followed by exsanguination by incision of the axillary or femoral arteries. All study animals were subjected to a limited necropsy examination, which consisted of an evaluation of the retained tissues. Representative samples of the conjunctiva, eyelids, harderian and lacrimal glands, extraocular muscles and nictitating membrane were collected from all animals and preserved in 10% neutral buffered formalin. The eyes and optic nerves were fixed in Davidson's fixative 24 to 48 hours, transferred to and stored in 70% ethanol for at least 18 hours before processing.

Five sagittal sections of each eye were prepared as follows: one section was taken at a target of 150 µm after the first exposure of tissue; one section at a target of 500 µm medially to the first one; two sections in the optic nerve separated by 100 to 300 µm; one section at least 500 µm medially of the optic nerve. All tissues were then embedded in paraffin, sectioned, mounted on glass slides, and stained with hematoxylin and eosin.

Histopathological evaluation was performed by a board-certified veterinary pathologist.

### 2. Results

Inflammation or dilatation of the tarsal gland of the eyelid was noted in a few animals in Group 1 and 2 (PBS and placebo), but not in Group 3 (ML7 0.1%), indicating an anti-inflammatory effect of ML7.

## Claims

1. A compound selected in the group consisting of ML7 or a derivative thereof, for use in a method for the treatment of an inflammation and/or dilatation of the tarsal gland of the eyelid.

2. The compound for use according to claim 1, which is to be administered in the form of a pharmaceutical composition further comprising a pharmaceutically acceptable carrier.

3. The compound for use according to claim 2, wherein the pharmaceutical composition is for topical administration to the eye.

4. The compound for use according to claim 3, wherein the pharmaceutical composition is an eye drop composition, in particular a multi dose eye drop composition.

5. The compound for use according to claim 2 to 4, wherein the composition comprises:
- between 0,001 and 0,5 weight % of the compound;
- between 0,1 and 10 weight % of cyclodextrine; and
- between 0,01 and 0,5 weight % of hydroxypropylmethylcellulose, PVP hydroxyethylcellulose or PVA.

6. The compound for use according to claim 5, wherein the cyclodextrine is hydroxypropyl beta-cyclodextrine.

7. The compound for use according to any one of claim 1 to 6, wherein the compound is ML7 or ML9.
